# FASCICULE DE BREVET EUROPEEN

(11) **EP 2 768 440 B1**
(45) Date de publication et mention de la délivrance du brevet: **06.01.2016**
(21) Numéro de dépôt: 12772935.8
(22) Date de dépôt: 12.10.2012
(51) Int. Cl.: A61F 5/01, A61H 3/00, B25J 9/00

(54) **EXOSQUELETTE INFERIEUR**
UNTERES EXOSKELETT
LOWER EXOSKELETON

(30) Priorité: 17.10.2011 FR 1159359
(43) Date de publication de la demande: 27.08.2014
(73) Titulaire: Commissariat à l'Énergie Atomique et aux Énergies Alternatives, 75015 Paris (FR); Robotiques 3 Dimensions, 89470 Monéteau (FR)
(72) Inventeur: GARREC, Philippe, 91190 Gif sur Yvette (FR); COSTE, Flavien, 51700 Champvoisy (FR); GRYGOROWICZ, Serge, 89580 Gy l'Évêque (FR); PERROT, Yann, 91700 Sainte-Geneviève des Bois (FR); PONSORT, Dominique, 91570 Bièvres (FR); RIGLET, Aurélie, 45230 Dammarie sur Loing (FR)
(74) Mandataire: Parzy, Benjamin Alain
(86) Numéro de dépôt international: PCT/EP2012/070333
(87) Numéro de publication internationale: WO 2013/057057

(56) Documents cités:
- GB-A- 2 260 495
- US-A1- 2006 064 047
- US-A1- 2006 260 620

## Description

L'invention est relative à un exosquelette, plus précisément un exosquelette inférieur s'étendant au niveau des jambes de l'utilisateur.

Dans tout ce qui suit, les orientations mentionnées des axes et des plans s'entendent alors que l'exosquelette est porté par l'utilisateur, les membres inférieurs de celui-ci étant au repos.

### ARRIERE-PLAN DE L'INVENTION

On connaît du document US2006/0260620 A1 un exosquelette inférieur destiné à être porté par un utilisateur, et comportant :
- un bassin pourvu de moyens de solidarisation à une partie supérieure du corps de l'utilisateur ;
- deux supports inférieurs pourvus de moyens de solidarisation aux membres inférieurs de l'utilisateur et reposant sur le sol durant les phases de station debout, les supports comportant chacun un segment de cuisse et un segment de tibia reliés par une liaison de genou configurée pour permettre une flexion et une extension entre le segment de cuisse et le segment de tibia ;
- des liaisons de hanche pour relier les deux supports inférieurs au bassin.

Dans ce document, le bassin se présente selon diverses variante :
- en une partie articulée selon un axe longitudinal (axe horizontal contenu dans un plan sagittal) sur un organe de solidarisation à l'utilisateur (une ceinture, un support dorsal...), pour autoriser des mouvements d'abduction et d'adduction des supports inférieurs. Il est à noter que dans cette configuration, l'abduction d'un support inférieur entraîne nécessairement l'adduction de l'autre support inférieur ;
- en deux parties articulées selon des axes longitudinaux parallèles sur un organe de solidarisation à l'utilisateur, pour autoriser des mouvements indépendants d'abduction et d'adduction des supports inférieurs ;
- en deux parties reliées à l'organe de solidarisation par des charnières élastiques pour autoriser des mouvements d'abduction et d'adduction des supports inférieurs de façon indépendante pour chacun des supports.

Il a été constaté que de tels bassins ne permettent pas toujours de suivre au mieux les mouvements naturels des membres inférieurs de l'utilisateur.

### OBJET DE L'INVENTION

L'invention vise à proposer un exosquelette inférieur dans lequel les segments de cuisse et de jambe suivent plus facilement les mouvements naturels des membres inférieurs de l'utilisateur.

### RESUME DE L'INVENTION

En vue de la réalisation de ce but, on propose un exosquelette inférieur destiné à être porté par un utilisateur, et comportant :
- un bassin équipé de moyens de solidarisation à une partie supérieure du corps de l'utilisateur ;
- deux supports inférieurs équipés de moyen de solidarisation aux membres inférieurs de l'utilisateur et reposant sur le sol durant les phases de station debout, les supports comportant chacun un segment de cuisse et un segment de tibia reliés par une liaison de genou configurée pour permettre une flexion et une extension entre le segment de cuisse et le segment de jambe ;
- des liaisons de hanche pour relier les deux supports inférieurs au bassin.

Selon l'invention, le bassin comporte :
- un segment interne s'étendant symétriquement par rapport à un plan sagittal médian et associé aux moyens de solidarisation à l'utilisateur;
- deux segments externes s'étendant symétriquement en étant reliés à des extrémités distales respectives du segment interne par l'intermédiaire d'organes de liaison laissant libre au moins des flexions entre le segment interne et chaque segment externe selon deux axes sensiblement orthogonaux;
chaque support inférieur étant relié par l'une des liaisons de hanche à l'un des segments externes.

Ainsi, les organes de liaison interposés entre le segment interne et les segments externes offrent des possibilités de déformation du bassin qui permettent à l'exosquelette de mieux suivre les mouvements naturels des membres inférieurs de l'utilisateur.

Selon un premier mode de réalisation de l'invention, chaque organe de liaison comprend un segment intermédiaire ayant une extrémité proximale reliée à une extrémité distale du segment interne par une liaison pivot selon un axe vertical, et une extrémité distale reliée à une extrémité proximale du segment externe associé par une liaison pivot selon un axe horizontal. La liaison ainsi construite s'apparente à une liaison de type Cardan.

Ces liaisons pivot seront de préférence pourvues d'organes de régulation, tels que des ressorts, des dissipateurs, voire des actionneurs si ces liaisons doivent être commandées.

Selon un deuxième mode de réalisation de l'invention, chaque organe de liaison comprend une lame déformable s'étendant selon un plan sensiblement vertical et ayant une extrémité proximale encastrée sur une extrémité distale du segment interne, et une extrémité distale encastrée sur une extrémité proximale du segment externe associé. La lame déformable peut fléchir et se tordre. La flexion de la lame assure la flexion entre le segment interne et chaque segment externe selon un axe vertical, tandis que la torsion de la lame assure la flexion entre le segment interne et chaque segment externe selon un axe horizontal.

Bien entendu, le segment interne pourra être décomposé en deux demi-segments reliés tous deux à un organe de solidarisation à l'utilisateur.

### BREVE DESCRIPTION DES FIGURES

L'invention sera mieux comprise à la lumière de la description qui suit de modes particuliers de réalisation de l'invention, en référence aux figures des dessins annexés parmi lesquels :
- la figure 1 est une vue en perspective schématique d'un exosquelette inférieur selon un premier mode particulier de l'invention, seule la partie droite de l'exosquelette ayant été représentée, étant entendu que l'exosquelette est symétrique par rapport au plan médian sagittal P ;
- la figure 2 est une vue schématique partielle du bassin de l'exosquelette de l'invention, selon un deuxième mode de réalisation de l'invention ;
- la figure 3 est une vue schématique de dessus du bassin de la figure 2 ;
- la figure 4 est une vue en perspective d'un exemple de réalisation du bassin des figures 2 et 3.

### DESCRIPTION DETAILLEE

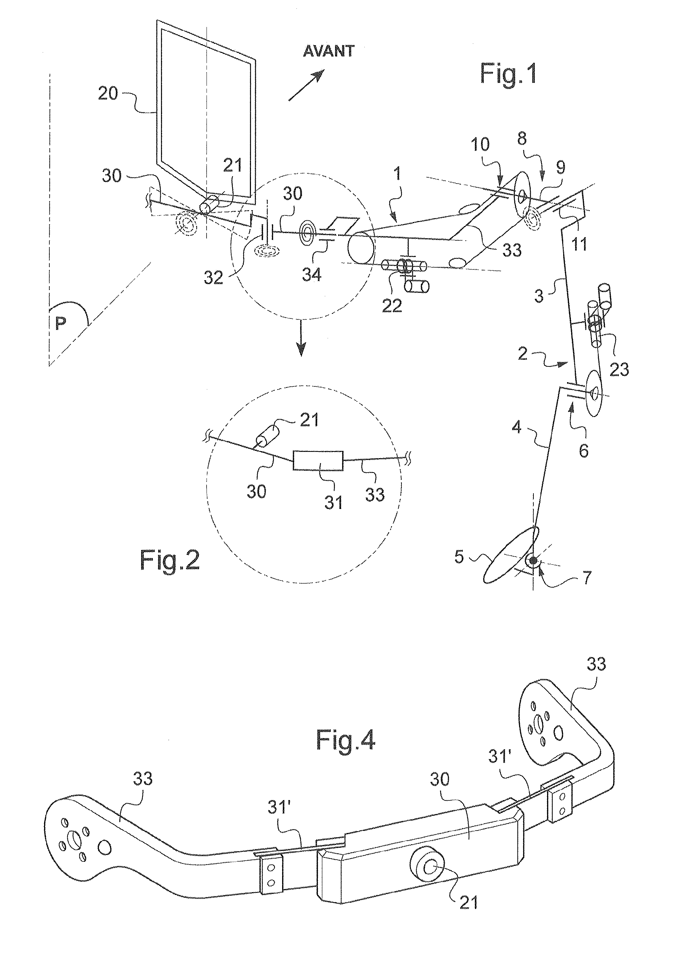

En référence à la figure 1, l'exosquelette inférieur de l'invention est destiné à équiper un utilisateur. A cet effet, il comporte une structure dorsale 20 destinée à être fixée dans le dos de l'utilisateur, par exemple au moyen d'une ceinture et de bretelles. L'exosquelette comporte un bassin 1 qui est ici articulé en son centre à la structure dorsale 20 par une liaison pivot 21 d'axe horizontal longitudinal (c'est-à-dire un axe horizontal s'étendant dans un plan sagittal), auquel sont reliés deux supports inférieurs 2 (un seul ici est visible) comportant chacun un segment de cuisse 3, un segment de tibia 4, et un pied 5.

Une liaison de genou 6, ici une liaison pivot d'axe horizontal transverse (c'est-à-dire perpendiculaire au plan sagittal médian P), relie le segment de cuisse 3 au segment de jambe 4, tandis qu'une liaison de cheville 7, ici une rotule, relie le segment de tibia au pied 5. Par ailleurs, chaque segment de cuisse 3 est relié au bassin 1 par une liaison de hanche 8 qui comporte un organe de liaison intermédiaire 9 relié d'une part au bassin 1 par une liaison pivot 10 d'axe horizontal transverse, et d'autre part au segment de cuisse 3 par une liaison pivot 11 d'axe horizontal longitudinal.

Bien sûr, les liaisons de genou, de cheville et de hanche pourront prendre toute autre conformation, sans pour autant faire sortir l'exosquelette du cadre de l'invention.

Ici, la liaison pivot 10 de la liaison de hanche 8 est associée à un actionneur 22 pour commander un mouvement entre le bassin 1 et l'organe de liaison intermédiaire 9. L'actionneur 22 est ici un actionneur à câble à double effet. On reviendra plus loin sur la façon dont cet actionneur est installé sur le bassin 1. En outre, la liaison de genou 6 est associée à un actionneur 23 disposé sur le segment de cuisse 3 pour commander un mouvement entre le segment de tibia 4 et le segment de cuisse 3. Les autres liaisons pivot sont ici équipées de ressorts de rappel vers une position d'équilibre.

Selon un aspect essentiel de l'invention, le bassin n'est pas monobloc, mais comprend :
- un segment interne 30 directement articulé sur la structure dorsale, et symétrique par rapport au plan sagittal médian P,
- deux segments intermédiaires 31 (un seul est visible ici) ayant une extrémité proximale liée par une liaison pivot 32 d'axe vertical à une extrémité distale du segment interne 30 ;
- deux segments externes 33 (un seul est visible ici) ayant une extrémité proximale liée par une liaison pivot 34 d'axe horizontal concourant avec l'axe vertical de la liaison pivot 32 à une extrémité distale d'un des segments intermédiaires 31.

Les liaisons pivot 32 et 34 sont ici équipées de ressorts de rappel vers une position d'équilibre.

Les deux libertés ainsi introduites permettent au bassin de mieux suivre les mouvements naturels de l'utilisateur. On remarquera que les segments intermédiaires 31 agissent à la manière d'un cardan entre le segment interne 30 et chaque segment externe 32.

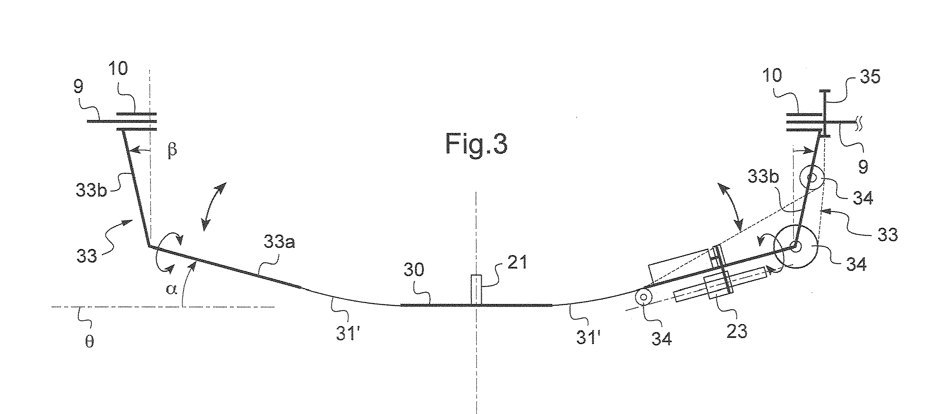

Comme cela est visible aux figure 2 à 4, et selon une variante de l'invention, les segments intermédiaires rigides 31 sont remplacés par des lames 31' déformables à la fois en flexion et en torsion. Chacune des lames 31' s'étend dans un plan vertical et est encastrée sur une extrémité distale du segment interne 30 et sur une extrémité proximale de l'un des segments externes 33. Comme cela est illustré à la figure 3 par les flèches en traits épais, les lames 31' sont déformables élastiquement (ou viscoélastiquement) à la fois en flexion et en torsion, de sorte que les lames 31' permettent les mêmes mouvements de segments externes 33 vis-à-vis du segment interne 30 que dans l'exemple précédent.

Selon un aspect particulier de l'invention, les segments externes 33 sont coudés, avec une extrémité proximale 33a s'étendant sensiblement dans le prolongement de l'extrémité distale associée du segment interne 30 en faisant un angle α avec un plan coronal Q de préférence compris entre 5 et 30 degrés, et une extrémité distale 33b s'étendant vers l'avant, en faisant un angle β avec un plan sagittal de préférence compris entre -10 et + 20 degrés.

L'actionneur à câble 23 est ici porté par l'extrémité proximale 33a du segment externe 33 derrière le bassin, des poulies de renvoi 34 permettant de renvoyer le câble (illustré en pointillés) vers une poulie 35 solidaire de l'organe de liaison intermédiaire 9 de la liaison de hanche 9. Bien sûr, et bien que non représenté, l'autre segment externe 33 porte également un actionneur à câble.

Ainsi, l'actionneur de commande de la liaison de hanche est logé dans le dos de l'utilisateur derrière le bassin, et peut être facilement intégré dans ce dernier. Le segment de cuisse est alors libéré d'un actionneur, ce qui contribue à diminuer le gabarit latéral de l'exosquelette inférieur de l'invention.

L'invention n'est pas limitée à ce qui vient d'être décrit, mais englobe au contraire toute variante entrant dans le cadre de l'invention défini par les revendications. En particulier, bien que dans l'exemple illustré, le segment interne du bassin soit monobloc, on pourra bien sûr utiliser un segment interne en deux parties, en s'inspirant par exemple du document US2006/0260620 A1. bien qu'ici les axes de flexion intermédiaire des bassins illustrés s'étendent selon des directions verticales et horizontales concourantes (axes des liaisons 32 et 34, axes de flexion et de torsion de la lame 31'), l'invention s'applique plus généralement à un bassin dont les segments externes sont reliés au segment interne par des organes de liaison laissant libre des flexions selon deux axes orthogonaux, concourants ou non.

## Revendications

1. Exosquelette inférieur destiné à être porté par un utilisateur, et comportant :
- un bassin (1) pourvu de moyens de solidarisation à une partie supérieure du corps de l'utilisateur ;
- deux supports inférieurs (2) pourvus de moyens de solidarisation aux membres inférieurs de l'utilisateur et reposant sur le sol durant les phases de station debout, les supports comportant chacun un segment de cuisse et un segment de tibia reliés par une liaison de genou configurée pour permettre une flexion et une extension entre le segment de cuisse et le segment de jambe ;
- des liaisons de hanche pour relier les deux supports inférieurs au bassin ;
- un segment interne (30) s'étendant symétriquement par rapport à un plan sagittal médian et associé aux moyens de solidarisation à l'utilisateur;
**caractérisé en ce que** le bassin comporte:
- deux segments externes (33) s'étendant symétriquement en étant reliés à des extrémités distales respectives du segment interne par l'intermédiaire d'organes de liaison (31, 32, 34 ; 31') laissant libre au moins des flexions entre le segment interne et chaque segment externe selon deux axes orthogonaux; chaque support inférieur étant relié par l'une des liaisons de hanche à l'un des segments externes (33).

2. Exosquelette inférieur selon la revendication 1, dans lequel chaque organe de liaison comprend un segment intermédiaire (31) ayant une extrémité proximale reliée à une extrémité distale du segment interne par une liaison pivot (32) selon un axe sensiblement vertical, et une extrémité distale reliée à une extrémité proximale du segment externe associé par une liaison pivot (34) selon un axe sensiblement horizontal.

3. Exosquelette inférieur selon la revendication 2, dans lequel les axes des liaisons pivot sont concourants.

4. Exosquelette inférieur selon la revendication 1, dans lequel chaque organe de liaison comprend une lame (31') déformable en flexion et en torsion s'étendant selon un plan sensiblement vertical et ayant une extrémité proximale encastrée sur une extrémité distale du segment interne, et une extrémité distale encastrée sur une extrémité proximale du segment externe associé.

5. Exosquelette inférieur selon la revendication 2, dans lequel chaque articulation de hanche comporte au moins une liaison pivot (10) commandée par un actionneur (22) disposé sur le bassin.

6. Exosquelette inférieur selon la revendication 5, dans lequel l'actionneur est solidaire du segment externe (33) portant l'articulation de hanche.

7. Exosquelette inférieur selon la revendication 6, dans lequel le segment externe est coudé, l'actionneur étant porté par une partie proximale du segment externe reliée au segment interne.

## Patentansprüche

1. Unteres Exoskelett, das dazu bestimmt ist, von einem Benutzer getragen zu werden, umfassend:
- ein Becken (1), das mit Mitteln zur Befestigung an einem oberen Teil des Körpers des Benutzers versehen ist;
- zwei untere Stützen (2), die mit Mitteln zur Befestigung an unteren Gliedmaßen des Benutzers versehen sind und während der Stehphasen auf dem Boden aufliegen, wobei die Stützen jeweils ein Oberschenkelsegment und ein Schienbeinsegment umfassen, die über eine Kniegelenkverbindung verbunden sind, die so konfiguriert ist, dass sie eine Beugung und eine Streckung zwischen dem Oberschenkelsegment und dem Unterschenkelsegment gestattet;
- Hüftverbindungen, um die beiden unteren Stützen mit dem Becken zu verbinden;
- ein Innensegment (30), das sich symmetrisch in Bezug auf eine Sagittalmittelebene erstreckt und mit Mitteln zur Befestigung am Benutzer verbunden ist;
**dadurch gekennzeichnet, dass** das Becken umfasst:
- zwei Außensegmente (33), die sich symmetrisch erstrecken und dabei mit jeweiligen distalen Enden des Innensegments durch Verbindungselemente (31, 32, 34; 31') verbunden sind, die zumindest Beugungen zwischen dem Innensegment und jedem Außensegment gemäß zwei orthogonalen Achsen gestatten,
wobei jede untere Stütze durch eine der Hüftverbindungen mit einem der Außensegmente (33) verbunden ist.

2. Unteres Exoskelett nach Anspruch 1, wobei jedes Verbindungselement ein Zwischensegment (31) umfasst, das ein proximales Ende hat, das mit einem distalen Ende des Innensegments über eine Drehverbindung (32) gemäß einer im Wesentlichen vertikalen Achse verbunden ist, sowie ein distales Ende, das mit einem proximalen Ende des Außensegments verbunden ist, das über eine Drehverbindung (34) gemäß einer im Wesentlichen horizontalen Achse verbunden ist.

3. Unteres Exoskelett nach Anspruch 2, wobei sich die Achsen der Drehverbindungen schneiden.

4. Unteres Exoskelett nach Anspruch 1, wobei jedes Verbindungselement ein auf Biegung und Torsion verformbares Plättchen (31') umfasst, das sich in einer im Wesentlichen vertikalen Ebene erstreckt und ein proximales Ende hat, das an einem distalen Ende des Innensegments eingefügt ist, sowie ein distales Ende, das an einem proximalen Ende des dazugehörigen Außensegments eingefügt ist.

5. Unteres Exoskelett nach Anspruch 2, wobei jede Hüftgelenkverbindung mindestens eine Drehverbindung (10) umfasst, die von einem Aktor (22) gesteuert wird, der auf dem Becken angeordnet ist.

6. Unteres Exoskelett nach Anspruch 5, wobei der Aktor fest mit dem Außensegment (33) verbunden ist, das die Hüftgelenkverbindung trägt.

7. Unteres Exoskelett nach Anspruch 6, wobei das Außensegment abgewinkelt ist, wobei der Aktor von einem proximalen Teil des Außensegments getragen wird, der mit dem Innensegment verbunden ist.

## Claims

1. A lower exoskeleton intended to be worn by a user and having:
- a pelvic element (1) provided with means for securing to an upper part of the body of the user;
- two lower supports (2) which are provided with means for securing to the lower limbs of the user and which rest on the ground during the stance phases, each of the supports having a thigh segment and a tibia segment which are connected by a knee link configured to allow flexion and extension between the thigh segment and the leg segment;
- hip links for connecting the two lower supports to the pelvic element;
- an inner segment (30) extending symmetrically in relation to a central sagittal plane and associated with the means for securing to the user;
**characterized in that** the pelvic element has:
- two outer segments (33) extending symmetrically and being connected to respective distal ends of the inner segment by means of linking members (31, 32, 34; 31'), allowing at least flexion between the inner segment and each outer segment along two orthogonal axes;
each lower support being connected by one of the hip links to one of the outer segments (33).

2. The lower exoskeleton as claimed in claim 1, in which each linking member comprises an intermediate segment (31) having a proximal end connected to a distal end of the inner segment by a pivot link (32) along a substantially vertical axis, and a distal end connected to a proximal end of the associated outer segment by a pivot link (34) along a substantially horizontal axis.

3. The lower exoskeleton as claimed in claim 2, in which the axes of the pivot links are concurrent.

4. The lower exoskeleton as claimed in claim 1, in which each linking member comprises a blade (31') that is deformable in flexion and in torsion and that extends along a substantially vertical plane and has a proximal end engaged on a distal end of the inner segment, and a distal end engaged on a proximal end of the associated outer segment.

5. The lower exoskeleton as claimed in claim 2, in which each hip joint has at least one pivot link (10) controlled by an actuator (22) arranged on the pelvic element.

6. The lower exoskeleton as claimed in claim 5, in which the actuator is rigidly connected to the outer segment (33) carrying the hip joint.

7. The lower exoskeleton as claimed in claim 6, in which the outer segment is bent, the actuator being carried by a proximal part of the outer segment connected to the inner segment.
